# EUROPEAN PATENT SPECIFICATION

(11) **EP 3 955 882 B1**
(45) Date of publication and mention of the grant of the patent: **23.04.2025**
(21) Application number: 20722811.5
(22) Date of filing: 06.04.2020
(51) Int. Cl.: A61K 8/29, A61K 8/28, A61K 8/898, A61K 8/891, A61K 8/58, A61Q 5/00

(54) **AN AMINOSILOXANE/ALKYL SILANE BLEND**
EINE AMINOSILOXAN/ALKYLSILAN-MISCHUNG
UN MÉLANGE D'AMINOSILOXANE/ALKYL SILANE

(30) Priority: 18.04.2019 US 201962835666 P
(43) Date of publication of application: 23.02.2022
(73) Proprietor: Dow Global Technologies LLC, Midland, MI 48674 (US)
(72) Inventor: ALMEIDA, Daniel, 04794-000 São Paulo - Sp (BR)
(74) Representative: Houghton, Mark Phillip
(86) International application number: PCT/US2020/026812
(87) International publication number: WO 2020/214442

(56) References cited:
- EP-A1- 0 113 992
- US-A- 4 344 763

## Description

The present invention relates to an aminosiloxane/alkyl silane blend, as defined in claim 1, for imparting hydrophobicity to hair. In particular, the present invention relates to an aminosiloxane/alkyl silane blend for imparting hydrophobicity to hair, comprising: 0.2 to 15 wt% of a cosmetically acceptable condensation catalyst selected from the group consisting of organic titanates, organic zirconates and mixtures thereof; 1 to 20 wt% of a cosmetically acceptable aminosiloxane of formula (I); 5 to 75 wt% of a cosmetically acceptable alkyl silane of formula (II); and 15 to 93.8 wt% of a cosmetically acceptable polydimethylsiloxane of formula (III).

Undamaged hair tends to be smooth and shiny and hydrophobic in nature preventing excessive water adsorption during washing. Once hair is damaged either mechanically or chemically (i.e., through bleaching, perming or coloring), the hair surface becomes more hydrophilic. As the hair surface becomes more hydrophilic, the resulting hair fibers tend to swell during washing making the hair more difficult to manage (e.g., comb).

There is an increasing demand for hair care products designed to retain the properties of undamaged hair and to help prevent damage. See e.g. EP 0 113 992, A1, (DOW CORNING [US]), 25 July 1984 (1984-07-25), and US 4 344 763, A, (TOLGYESI EVA ET AL), 17 August 1982 (1982-08-17).

There remains a continuing need for additives and blends for use in personal care formulations (in particular hair care formulations) that impart hydrophobicity to hair.

The present invention provides a aminosiloxane/alkyl silane blend for imparting hydrophobicity to hair, comprising: (a) 0.2 to 15 wt% of a cosmetically acceptable condensation catalyst selected from the group consisting of organic titanates, organic zirconates and mixtures thereof; (b) 1 to 20 wt% of a cosmetically acceptable aminosiloxane of formula (I) 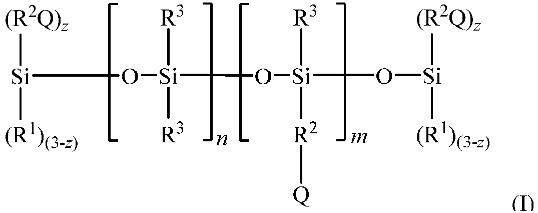 wherein each R¹ is independently selected from the group consisting of a C₁₋₃₀ alkyl group, a C₁₋₃₀ alkoxy group, a C₅₋₃₀ aryl group, a C₆₋₃₀ aralkyl group, a C₆₋₃₀ aralkyloxy group, a C₁₋₃₀ alkaryl group, a C₁₋₃₀ alkoxyaryl group, and a hydroxy group; wherein R² is a divalent alkylene radical having one to ten carbon atoms; wherein each R³ is independently selected from the group consisting of a C₁₋₃₀ alkyl group, a C₅₋₃₀ aryl group, a C₆₋₃₀ aralkyl group and a C₁₋₃₀ alkaryl group; wherein Q is a monovalent radical selected from the group consisting of -NR⁴₂ and -NR⁴(CH₂)*ₓ*NR⁴₂; wherein each R⁴ is independently selected from the group consisting of a hydrogen and a C₁₋₄ alkyl group; wherein x is 2 to 6; wherein *z* is 0 or 1; wherein *n* is 25 to 3,000 and wherein *m* is 0 to 3,000; with the proviso that at least 50 mol% of the total number of R¹ and R³ groups are methyl and with the proviso that when m is 0, *z* is 1; (c) 5 to 75 wt% of a cosmetically acceptable alkyl silane of formula (II) 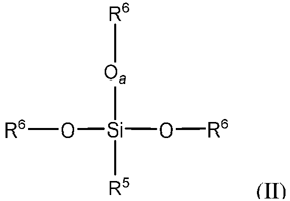 wherein *a* is selected from 0 and 1; wherein R⁵ is a C₂₋₁₀ alkyl group with an addition reaction functional moiety selected from epoxide group and vinyl group and wherein each R⁶ is independently selected from a C₁₋₄ alkyl group; and (d) 15 to 93.8 wt% of a cosmetically acceptable polydimethylsiloxane of formula (III) 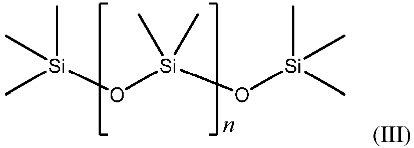 wherein *n* is an average of 0 to 150.

### DETAILED DESCRIPTION

We have now surprisingly found that the identified unique blend of four components provides exemplary persistent hydrophobicity to damaged hair, wherein the treated hair exhibits continued enhanced hydrophobicity after as many as thirty subsequent washes.

Unless otherwise indicated, ratios, percentages, parts, and the like are by weight.

The term "cosmetically acceptable" as used herein and in the appended refers to ingredients typically used in personal care compositions, and is intended to underscore that materials that are toxic when present in the amounts typically found in personal care compositions are in hair care compositions are not contemplated as part of the present invention.

Preferably, the aminosiloxane/alkyl silane blend for imparting hydrophobicity to hair of the present invention, comprises: (a) 0.2 to 15 wt% (preferably, 0.5 to 10 wt%; more preferably, 1 to 8 wt%) of a cosmetically acceptable condensation catalyst selected from the group consisting of organic titanates, organic zirconates and mixtures thereof; (b) 1 to 20 wt% (preferably, 1.5 to 17 wt%; more preferably, 2 to 15 wt%) of a cosmetically acceptable aminosiloxane of formula (I) 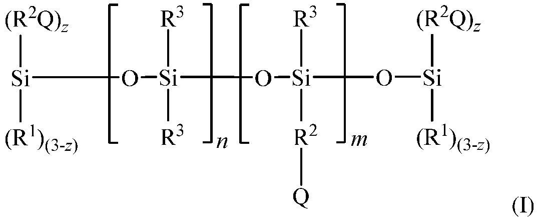 wherein each R¹ is independently selected from the group consisting of a C₁₋₃₀ alkyl group, a C₁₋₃₀ alkoxy group, a C₅₋₃₀ aryl group, a C₆₋₃₀ aralkyl group, a C₆₋₃₀ aralkyloxy group, a C₁₋₃₀ alkaryl group, a C₁₋₃₀ alkoxyaryl group, and a hydroxy group; wherein R² is a divalent alkylene radical having one to ten carbon atoms (preferably, three to six carbon atoms); wherein each R³ is independently selected from the group consisting of a C₁₋₃₀ alkyl group, a C₅₋₃₀ aryl group, a C₆₋₃₀ aralkyl group and a C₁₋₃₀ alkaryl group; wherein Q is a monovalent radical selected from the group consisting of -NR⁴₂ and -NR⁴(CH₂)*ₓ*NR⁴₂; wherein each R⁴ is independently selected from the group consisting of a hydrogen and a C₁₋₄ alkyl group; wherein *x* is 2 to 6; wherein *z* is 0 or 1; wherein *n* is 25 to 3,000 (preferably, 25 to 2,000; more preferably, 25 to 1,000; most preferably 25 to 500) and wherein m is 0 to 3,000 (preferably, 0 to 2,000; more preferably, 0 to 1,000; most preferably, 0 to 100); with the proviso that at least 50 mol% of the total number of R¹ and R³ groups are methyl and with the proviso that when m is 0, *z* is 1; (c) 5 to 75 wt% (preferably, 10 to 70 wt%; more preferably, 40 to 60 wt%) of a cosmetically acceptable alkyl silane of formula (II) 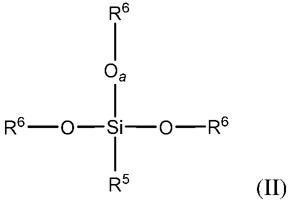 wherein *a* is selected from 0 and 1 (preferably, wherein *a* is a 1); wherein R⁵ is a C₂₋₁₀ alkyl group with an addition reaction functional moiety; and wherein each R⁶ is independently selected from a C₁₋₄ alkyl group; and (d) 15 to 93.8 wt% (preferably, 20 to 88 wt%; more preferably, 25 to 57 wt%) of a cosmetically acceptable polydimethylsiloxane of formula (III) 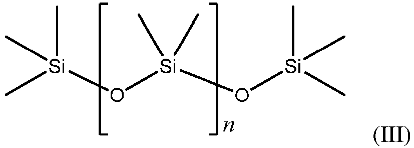 wherein *n* is an average of 0 to 150 (preferably, 0 to 100; more preferably, 0 to 50; still more preferably, 0 to 25; most preferably, 0 to 8).

Preferably, the aminosiloxane/alkyl silane blend for imparting hydrophobicity to hair of the present invention, comprises a cosmetically acceptable condensation catalyst selected from the group consisting of organic titanates, organic zirconates and mixtures thereof. More preferably, the aminosiloxane/alkyl silane blend for imparting hydrophobicity to hair of the present invention, comprises 0.2 to 15 wt% (preferably, 0.5 to 10 wt%; more preferably, 1 to 8 wt%) of a cosmetically acceptable condensation catalyst selected from the group consisting of organic titanates, organic zirconates and mixtures thereof. Most preferably, the aminosiloxane/alkyl silane blend for imparting hydrophobicity to hair of the present invention, comprises 0.2 to 15 wt% (preferably, 0.5 to 10 wt%; more preferably, 1 to 8 wt%) of a cosmetically acceptable condensation catalyst, wherein the cosmetically acceptable condensation catalyst includes an organic titanate (preferably, a tetra-n-butyl titanate).

Preferably, the organic titanate and organic zirconate based catalysts from which the cosmetically acceptable condensation catalyst is selected comprise compounds according to the general formula M[OR]₄; wherein M is selected from the group consisting of Ti and Zr (preferably, Ti) and wherein each R is independently selected from a monovalent, primary, secondary or tertiary aliphatic hydrocarbon group which can be linear or branched containing 1 to 10 carbon atoms. Preferably, each R is independently selected from the group consisting of a methyl group, an ethyl group, a propyl group, an isopropyl group, a butyl group, a tertiary butyl group and a branched secondary alkyl group (e.g., 2,4-dimethyl-3-pentyl). More preferably, all the R's are the same. Yet more preferably, all the R's are the same and are selected from n-butyl, t-butyl, butyl and isopropyl. Most preferably, all the R's are the same and are n-butyl.

Preferably, the aminosiloxane/alkyl silane blend for imparting hydrophobicity to hair of the present invention, comprises a cosmetically acceptable aminosiloxane of formula (I). More preferably, the aminosiloxane/alkyl silane blend for imparting hydrophobicity to hair of the present invention, comprises 1 to 20 wt% (preferably, 1.5 to 17 wt%; more preferably, 2 to 15 wt%) of a cosmetically acceptable aminosiloxane of formula (I) 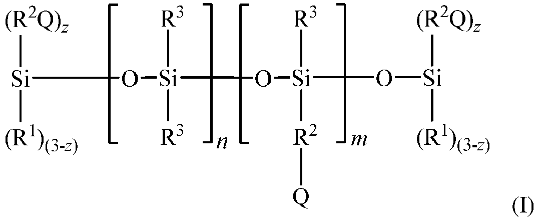 wherein each R¹ is independently selected from the group consisting of a C₁₋₃₀ alkyl group, a C₁₋₃₀ alkoxy group, a C₅₋₃₀ aryl group, a C₆₋₃₀ aralkyl group, a C₆₋₃₀ aralkyloxy group, a C₁₋₃₀ alkaryl group, a C₁₋₃₀ alkoxyaryl group, and a hydroxy group (preferably, wherein each R¹ is independently selected from the group consisting of a C₁₋₃₀ alkyl group, a C₁₋₃₀ alkoxy group and a hydroxy group); wherein R² is a divalent alkylene radical having one to ten carbon atoms (preferably, wherein R² is a divalent alkylene radical having three to six carbon atoms); wherein each R³ is independently selected from the group consisting of a C₁₋₃₀ alkyl group, a C₅₋₃₀ aryl group, a C₆₋₃₀ aralkyl group and a C₁₋₃₀ alkaryl group (preferably, wherein each R³ is independently selected from the group consisting of a C₁₋₃₀ alkyl group); wherein Q is a monovalent radical selected from the group consisting of -NR⁴₂ and -NR⁴(CH₂)*ₓ*NR⁴₂; wherein each R⁴ is independently selected from the group consisting of a hydrogen and a C₁₋₄ alkyl group; wherein *x* is 2 to 6; wherein *z* is 0 or 1; wherein *n* is 25 to 3,000 (preferably, 25 to 2,000; more preferably, 25 to 1,000; most preferably 25 to 500) and wherein m is 0 to 3,000 (preferably, 0 to 2,000; more preferably, 0 to 1,000; most preferably, 0 to 100); with the proviso that at least 50 mol% of the total number of R¹ and R³ groups are methyl and with the proviso that when m is 0, *z* is 1.

Preferred R¹ groups include methyl, methoxy, ethyl, ethoxy, propyl, propoxy, isopropyl, isopropoxy, butyl, butoxy, isobutyl, isobutoxy, phenyl, xenyl, benzyl, phenylethyl, tolyl and hydoxy.

Preferred R² divalent alkylene radicals include trimethylene, tetramethylene, pentamethylene, -CH₂CH(CH₃)CH₂- and -CH₂CH₂CH(CH₃)CH₂-.

Preferred R³ groups include methyl, ethyl, propyl, isopropyl, butyl, isobutyl, phenyl, xenyl, benzyl, phenylethyl and tolyl.

Preferred R⁴ groups include methyl, ethyl, propyl, isopropyl, butyl and isobutyl.

When *z* is 0, the cosmetically acceptable aminosiloxane has only pendant amine functional substituents in the polymer chain. When *z* is 1, the cosmetically acceptable aminosiloxane may have only terminal amine functional substituents (e.g., *m*=0) or may have both terminal and pendant amine functional substituents in the polymer chain (e.g., *m>0).*

Preferably, *n + m* is 50 to 1,000. More preferably, *n + m* is 50 to 750. Still more preferably, *n + m* is 50 to 500. Most preferably, *n + m* is 50 to 250.

Preferably, the aminosiloxane/alkyl silane blend for imparting hydrophobicity to hair of the present invention, comprises a cosmetically acceptable alkyl silane of formula (II). More preferably, the aminosiloxane/alkyl silane blend for imparting hydrophobicity to hair of the present invention, comprises 5 to 75 wt% (preferably, 10 to 70 wt%; more preferably, 40 to 60 wt%) of a cosmetically acceptable alkyl silane of formula (II) 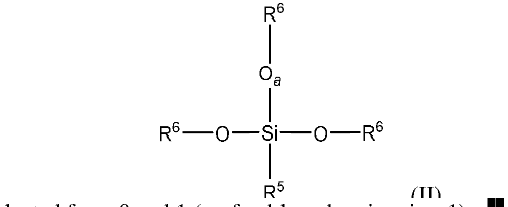 wherein *a* is selected from 0 and 1 (preferably, wherein *a* is a 1); wherein R⁵ is a C₂₋₁₀ alkyl group with an addition reaction functional moiety selected from an epoxide group (e.g., glycidyl) and a vinyl group); and wherein each R⁶ is independently selected from a C₁₋₄ alkyl group; (preferably, wherein each R⁶ is independently selected from a C₁₋₃ alkyl group; more preferably, wherein each R⁶ is independently selected from a C₁₋₂ alkyl group; most preferably, wherein each R⁶ is an ethyl group).

Optionally, the R⁵ group can be further substituted with an oxygen atom. For example, the R⁵ group can include an oxygen forming an ether linkage within the C₂₋₁₀ alkyl group.

Optionally, the R⁵ group is selected from a C₂₋₁₀ alkyl group, wherein the C₂₋₁₀ alkyl group can include a cyclic structure (e.g., a cyclohexyl ring).

Preferably, the aminosiloxane/alkyl silane blend for imparting hydrophobicity to hair of the present invention, comprises a cosmetically acceptable polydimethylsiloxane of formula (III). More preferably, the aminosiloxane/alkyl silane blend for imparting hydrophobicity to hair of the present invention, comprises 15 to 93.8 wt% (preferably, 20 to 88 wt%; more preferably, 25 to 57 wt%) of a cosmetically acceptable polydimethylsiloxane of formula (III) 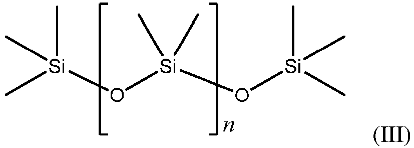 wherein *n* is an average of 0 to 150 (preferably, 0 to 100; more preferably, 0 to 50; still more preferably, 0 to 25; most preferably, 0 to 8).

Preferably, in preparing the aminosiloxane/alkyl silane blend for imparting hydrophobicity to hair of the present invention, the cosmetically acceptable polydimethylsiloxane of formula (III) is blended with the cosmetically acceptable alkyl silane of formula (II) and the cosmetically acceptable condensation catalyst with moderate agitation (e.g., mechanical stirring) until uniform. Then the cosmetically acceptable aminosiloxane of formula (I) is slowly added to the system with continued agitation until uniform. While not mandatory, it is preferred to prepare the aminosiloxane/alkyl silane blend in an inert atmosphere (e.g., nitrogen).

The aminosiloxane/alkyl silane blend for imparting hydrophobicity to hair of the present invention may be used as provided to treat hair. Alternatively, the aminosiloxane/alkyl silane blend for imparting hydrophobicity to hair of the present invention may be used as an additive for incorporating into a formulated hair care system to provide that system with the ability to impart hydrophobicity to hair.

Some embodiments of the present invention will now be described in detail in the following **Examples.**

### Comparative Examples C1-C2 and Examples 1-4: Aminosiloxane/alkyl silane blends

A blend was prepared having the composition of components noted in **TABLE 1** for each of **Comparative Examples C1-C2** and **Examples 1-4.**

**TABLE 1**

| **Component** | **Example (wt%)** | | | | | |
|---|---|---|---|---|---|---|
| | **Comp. Ex. C1** | **Comp. Ex. C2** | **Ex. 1** | **Ex. 2** | **Ex. 3** | **Ex. 4** |
| Condensation catalyst¹ | 6 | 6 | 6 | 6 | 6 | 6 |
| non-addition reactive alkyl silane² | 50 | -- | -- | -- | -- | -- |
| addition reactive alkyl silane³ | -- | -- | 50 | -- | -- | -- |
| addition reactive alkyl silane⁴ | -- | -- | -- | 50 | 50 | 50 |
| Aminosiloxane⁵ | -- | -- | -- | 10 | -- | -- |
| Aminosiloxane⁶ | -- | -- | -- | -- | 10 | -- |
| Aminosiloxane⁷ | 10 | 10 | 10 | -- | -- | 10 |
| Polydimethylsiloxane⁸ | 34 | 84 | 34 | 34 | 34 | 34 |
| ¹ Tyzor TnBT organic titanate (tetra-n-butyl titanate) available from Dorf Ketal. | | | | | | |
| ² Xiameter^{®} OFS-6381 alkyl functional silane available from The Dow Chemical Company. | | | | | | |
| ³ Xiameter^{®} OFS-6106 glycidoxi functional silane available from The Dow Chemical Company. | | | | | | |
| ⁴ Xiameter^{®} OFS-6518 vinyl functional silane available from The Dow Chemical Company | | | | | | |
| ⁵ DOWSIL^{™} 2-8566 conditioning agent (INCI: Amodimethicone) available from The Dow Chemical Company. | | | | | | |
| ⁶ DOWSIL^{™} 8500 conditioning agent (INCI: Bis (C₁₃₋₁₅ alkoxy) PG-Amodimethicone) available from The Dow Chemical Company. | | | | | | |
| ⁷ DOWSIL^{™} AP-8087 conditioning agent (INCI: Bis-Hydroxy/Methoxy Amodimethicone) available from The Dow Chemical Company. | | | | | | |
| ⁸ DOWSIL^{™} PMX 200/2 fluid (INCI: Dimethicone) available from The Dow Chemical Company. | | | | | | |

### Hair Hydrophobicity

The blends prepared according to each of **Comparative Examples C1-C2** and **Examples 1-4** were tested on two separate 3 g hair samples (Slightly Bleached Caucasian Hair, International Hair Importers, Inc.). The hair samples were first rinsed with water for 30 seconds. Then a 9% w/w aqueous solution of sodium lauryl ether sulfate was massaged into the hair samples for 30 seconds. Then the hair samples were rinsed with water for 30 seconds. Finally, the blends prepared according to **Comparative Examples C1-C2** and **Examples 1-4** were diluted with polydimethylsiloxane (DOWSIL^{™} PMX 200/2 fluid) to provide 20 wt% of each blend in PDMS. The diluted blends were then added to separate hair samples at the dosage (g of diluted blend/g of hair) as noted in TABLE 2 and massaged in to the hair for 30 seconds. The hair samples where then rinsed with water for 30 seconds and dried before hydrophobicity testing. The water used for rinsing in this testing was softened to a hardness of 80 ppm and heated to 38 °C.

To measure hydrophobicity of the hair, the tresses were combed straight and the tresses were held tightly on both ends with a holder. Ten drops of water were placed at different locations on each tress from the root to the tip. Measurements of the water contact angle at each drop were taken ten seconds after the drop of water got in contact with the tress. The higher the angle, the higher the hydrophobicity (which is desirable). This process was repeated for each of the tresses after washing 0, 5, 10, 20 and 30 times with a 9% w/w aqueous solution of sodium lauryl ether sulfate. The average contact angle is reported in **TABLE 2.**

**TABLE 2**

| | | **Contact angle (in degrees) following the noted number of washes** | | | | |
|---|---|---|---|---|---|---|
| **Blend** | **Dosage** | **0** | **5** | **10** | **20** | **30** |
| C1 | 0.15 | 100.5 | 13.7 | 35.7 | 0 | 0 |
| C1 | 0.30 | 94.9 | 91.2 | 35.7 | 17.9 | 0 |
| C2 | 0.15 | 121.7 | 7.4 | 0 | 0 | 0 |
| C2 | 0.30 | 130.2 | 98.3 | 65.3 | 0 | 0 |
| 1 | 0.15 | 31.0 | 111.5 | 101.4 | 102.4 | 70.9 |
| 1 | 0.30 | 107.0 | 117.2 | 109.0 | 110.3 | 84.3 |
| 2 | 0.15 | 123.6 | 110.4 | 93.3 | 91.7 | 65.6 |
| 2 | 0.30 | 121.7 | 112.5 | 90.9 | 95.9 | 42.4 |
| 3 | 0.15 | 131.1 | 112.1 | 73.1 | 41.1 | 23.8 |
| 3 | 0.30 | 122.7 | 111.2 | 104.5 | 93.0 | 33.7 |
| 4 | 0.15 | 128.9 | 108.6 | 93.3 | 40.6 | 43.7 |
| 4 | 0.30 | 132.9 | 114.9 | 102.9 | 100.3 | 45.6 |

### Hair Coefficient of Friction

Coefficient of friction is an industry standard method of measuring reduced frictional properties of treatments on hair and correlates with sensory attributes for smoothness and softness. A Diastron MTT175 tensile tester with 50 g normal force mounted on rubber probe was used for testing in a temperature and humidity controlled room. Two tresses per treatment and five measurements per tress were tested to generate the average friction data reported in **TABLE 3.** Coefficient of friction (COF) = F/N, wherein F was the externally applied force and N was the normal force. The same tresses from the hydrophobicity study were used for measuring the coefficient of friction in the dry state.

**TABLE 3**

| | | **COF following the noted number of washes** | | | | |
|---|---|---|---|---|---|---|
| **Blend** | **Dosage** | **0** | **5** | **10** | **20** | **30** |
| C1 | 0.15 | 0.29 | 0.63 | 0.62 | 0.57 | -- |
| C1 | 0.30 | 0.25 | 0.69 | 0.52 | 0.65 | -- |
| C2 | 0.15 | 0.68 | 0.65 | -- | -- | -- |
| C2 | 0.30 | 0.59 | 0.71 | 0.67 | -- | -- |
| 1 | 0.15 | 0.30 | 0.36 | 0.44 | 0.56 | 0.56 |
| 1 | 0.30 | 0.25 | 0.33 | 0.48 | 0.52 | 0.58 |
| 2 | 0.15 | 0.42 | 0.51 | 0.62 | 0.59 | 0.64 |
| 2 | 0.30 | 0.48 | 0.57 | 0.57 | 0.66 | 0.59 |
| 3 | 0.15 | 0.48 | 0.61 | 0.63 | 0.54 | 0.65 |
| 3 | 0.30 | 0.54 | 0.57 | 0.54 | 0.72 | 0.61 |
| 4 | 0.15 | 0.40 | 0.45 | 0.63 | 0.54 | 0.66 |
| 4 | 0.30 | 0.39 | 0.47 | 0.67 | 0.56 | 0.47 |

## Claims

1. An aminosiloxane/alkyl silane blend for imparting hydrophobicity to hair, comprising:
(a) 0.2 to 15 wt% of a cosmetically acceptable condensation catalyst selected from the group consisting of organic titanates, organic zirconates and mixtures thereof;
(b) 1 to 20 wt% of a cosmetically acceptable aminosiloxane of formula (I) 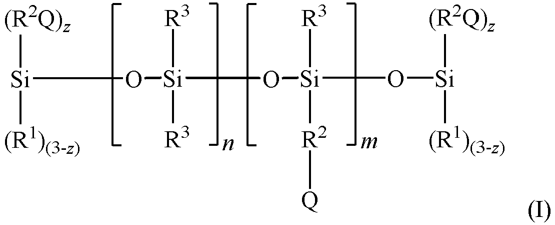
wherein each R¹ is independently selected from the group consisting of a C₁₋₃₀ alkyl group, a C₁₋₃₀ alkoxy group, a C₅₋₃₀ aryl group, a C₆₋₃₀ aralkyl group, a C₆₋₃₀ aralkyloxy group, a C₁₋₃₀ alkaryl group, a C₁₋₃₀ alkoxyaryl group, and a hydroxy group;
wherein R² is a divalent alkylene radical having one to ten carbon atoms;
wherein each R³ is independently selected from the group consisting of a C₁₋₃₀ alkyl group, a C₅₋₃₀ aryl group, a C₆₋₃₀ aralkyl group and a C₁₋₃₀ alkaryl group;
wherein Q is a monovalent radical selected from the group consisting of -NR⁴₂ and -NR⁴(CH₂)*ₓ*NR⁴₂;
wherein each R⁴ is independently selected from the group consisting of a hydrogen and a C₁₋₄ alkyl group;
wherein x is 2 to 6;
wherein z is 0 or 1;
wherein *n* is 25 to 3,000; and
wherein *m* is 0 to 3,000;
with the proviso that at least 50 mol% of the total number of R¹ and R³ groups are methyl and with the proviso that when *m* is 0, *z* is 1;
(c) 5 to 75 wt% of a cosmetically acceptable alkyl silane of formula (II) 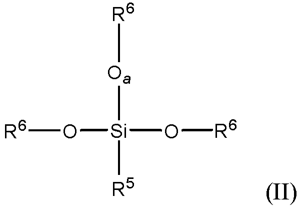
wherein *a* is selected from 0 and 1;
wherein R⁵ is a C₂₋₁₀ alkyl group with an addition reaction functional moiety selected from an epoxide group and a vinyl group; and
wherein each R⁶ is independently selected from a C₁₋₄ alkyl group; and
(d) 15 to 93.8 wt% of a cosmetically acceptable polydimethylsiloxane of formula (III) 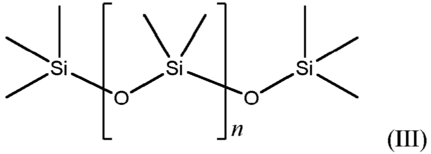 wherein *n* is an average of 0 to 150.

2. The aminosiloxane/alkyl silane blend of claim 1, wherein the addition reaction functional moiety is an epoxide group.

3. The aminosiloxane/alkyl silane blend of claim 1, wherein the cosmetically acceptable condensation catalyst includes a titantate.

4. The aminosiloxane/alkyl silane blend of claim 1, wherein z is 0.

5. The aminosiloxane/alkyl silane blend of claim 4, wherein R² is selected from the group consisting of trimethylene, tetramethylene,
pentamethylene, -CH₂CH(CH₃)CH₂- and -CH₂CH₂CH(CH₃)CH₂-.

6. The aminosiloxane/alkyl silane blend of claim 1, wherein *a* is 1.

7. The aminosiloxane/alkyl silane blend of claim 1, wherein the aminosiloxane/alkyl blend comprises:
1 to 8 wt% of the cosmetically acceptable condensation catalyst;
2 to 15 wt% of the cosmetically acceptable aminosiloxane of formula (I);
40 to 60 wt% of the cosmetically acceptable alkyl silane of formula (II); and
25 to 57 wt% of the cosmetically acceptable polydimethylsiloxane of formula (III).

8. The aminosiloxane/alkyl silane blend of claim 7, wherein the cosmetically acceptable condensation catalyst is a organic titanate; wherein z is 0 and wherein the addition reaction functional group is selected from the group consisting of an epoxide group and a vinyl group.

9. The aminosiloxane/alkyl silane blend of claim 8, wherein R² is selected from the group consisting of trimethylene, tetramethylene,
pentamethylene, -CH₂CH(CH₃)CH₂- and -CH₂CH₂CH(CH₃)CH₂-.

10. The aminosiloxane/alkyl silane blend of claim 9, wherein *a* is 1.

## Patentansprüche

1. Aminosiloxan-Alkylsilan-Mischung zum Hydrophobieren von Haaren, umfassend:
(a) zu 0,2 bis 15 Gew.-% einen kosmetisch verträglichen Kondensationskatalysator, ausgewählt aus der Gruppe bestehend aus organischen Titanaten, organischen Zirkonaten und Mischungen davon;
(b) zu 1 bis 20 Gew.-% ein kosmetisch akzeptables Aminosiloxan der Formel (I) 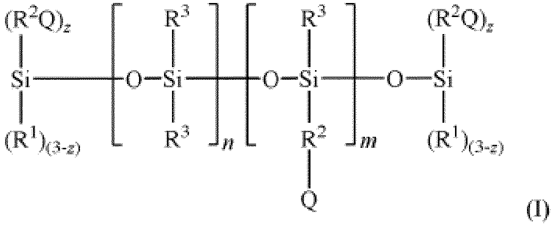
wobei jedes R¹ unabhängig ausgewählt ist aus der Gruppe bestehend aus einer C₁₋₃₀-Alkylgruppe, einer C₁₋₃₀-Alkoxygruppe, einer C₅₋₃₀-Arylgruppe, einer C₆₋₃₀-Aralkylgruppe, einer C₆₋₃₀-Aralkyloxygruppe, einer C₁₋₃₀-Alkarylgruppe, einer C₁₋₃₀-Alkoxyarylgruppe und einer Hydroxygruppe;
wobei R² ein zweiwertiger Alkylenrest mit ein bis zehn Kohlenstoffatomen ist;
wobei jedes R³ unabhängig ausgewählt ist aus der Gruppe bestehend aus einer C₁₋₃₀-Alkylgruppe, einer C₅₋₃₀-Arylgruppe, einer C₆₋₃₀-Aralkylgruppe und einer C₁₋₃₀-Alkarylgruppe;
wobei Q ein einwertiger Rest ist, ausgewählt aus der Gruppe bestehend aus -NR⁴₂ und -NR⁴(CH₂)*ₓ*NR⁴₂;
wobei jedes R⁴ unabhängig aus der Gruppe ausgewählt ist, bestehend aus einem Wasserstoff und einer C₁₋₄-Alkylgruppe;
wobei x 2 bis 6 ist;
wobei z 0 oder 1 ist;
wobei *n* 25 bis 3.000 beträgt; und
wobei m von 0 bis 3.000 ist;
mit der Maßgabe, dass mindestens 50 Mol-% der Gesamtzahl der R¹- und R³-Gruppen Methyl sind und mit der Maßgabe, dass wenn m gleich 0 ist, z gleich 1 ist;
(c) zu 5 bis 75 Gew.-% ein kosmetisch verträgliches Alkylsilan der Formel (II) 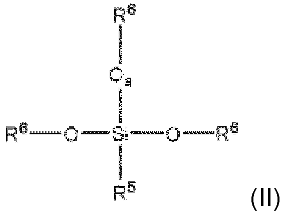
wobei a aus 0 und 1 ausgewählt ist;
wobei R⁵ eine C₂₋₁₀-Alkylgruppe mit einer funktionellen Additionsreaktionseinheit ist, ausgewählt aus einer Epoxidgruppe und einer Vinylgruppe; und
wobei jedes R⁶ unabhängig aus einer C₁₋₄-Alkylgruppe ausgewählt ist; und
(d) zu 15 bis 93,8 Gew.-% ein kosmetisch verträgliches Polydimethylsiloxan der Formel (III) 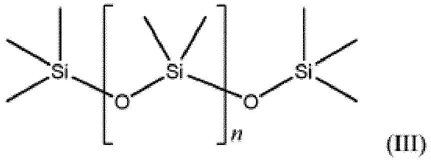 wobei *n* ein Durchschnitt von 0 bis 150 ist;

2. Aminosiloxan-Alkylsilan-Mischung nach Anspruch 1, wobei die funktionelle Additionsreaktionseinheit eine Epoxidgruppe ist.

3. Aminosiloxan-Alkylsilan-Mischung nach Anspruch 1, wobei der kosmetisch verträgliche Kondensationskatalysator ein Titantat einschließt.

4. Aminosiloxan-Alkylsilan-Mischung nach Anspruch 1, wobei z gleich 0 ist.

5. Aminosiloxan-Alkylsilan-Mischung nach Anspruch 4, wobei R² ausgewählt ist aus der Gruppe bestehend aus Trimethylen, Tetramethylen,
Pentamethylen, -CH₂CH(CH₃)CH₂- und -CH₂CH₂CH(CH₃)CH₂-.

6. Aminosiloxan-Alkylsilan-Mischung nach Anspruch 1, wobei a gleich 1 ist.

7. Aminosiloxan-Alkylsilan-Mischung nach Anspruch 1, wobei die Aminosiloxan-Alkyl-Mischung umfasst:
zu 1 bis 8 Gew.-% den kosmetisch verträglichen Kondensationskatalysator;
zu 2 bis 15 Gew.-% das kosmetisch verträgliche Aminosiloxan der Formel (I);
zu 40 bis 60 Gew.-% das kosmetisch verträgliche Alkylsilan der Formel (II); und
zu 25 bis 57 Gew.-% das kosmetisch verträgliche Polydimethylsiloxan der Formel (III).

8. Aminosiloxan-Alkylsilan-Mischung nach Anspruch 7, wobei der kosmetisch verträgliche Kondensationskatalysator ein organisches Titanat ist; wobei z gleich 0 ist und wobei die funktionelle Additionsreaktionsgruppe aus der Gruppe ausgewählt ist, die aus einer Epoxidgruppe und einer Vinylgruppe besteht.

9. Aminosiloxan-Alkylsilan-Mischung nach Anspruch 8, wobei R² ausgewählt ist aus der Gruppe bestehend aus Trimethylen, Tetramethylen,
Pentamethylen, -CH₂CH(CH₃)CH₂- und -CH₂CH₂CH(CH₃)CH₂-.

10. Aminosiloxan-Alkylsilan-Mischung nach Anspruch 9, wobei a gleich 1 ist.

## Revendications

1. Mélange d'aminosiloxane/alkylsilane permettant de conférer une hydrophobicité aux cheveux, comprenant :
(a) 0,2 à 15 % en poids d'un catalyseur de condensation cosmétiquement acceptable choisi dans le groupe constitué par les titanates organiques, les zirconates organiques et leurs mélanges ;
(b) 1 à 20 % en poids d'un aminosiloxane cosmétiquement acceptable de formule (I) 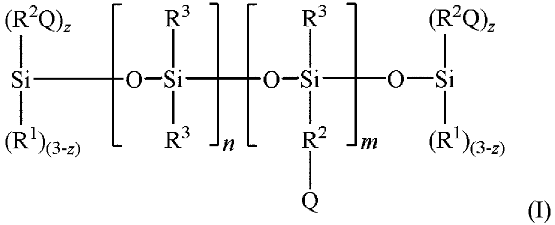
où chaque R¹ est indépendamment choisi dans le groupe constitué d'un groupe alkyle en C_{1 à 30}, d'un groupe alcoxy en C_{1 à 30}, d'un groupe aryle en C_{5 à 30}, d'un groupe aralkyle en C_{6 à 30}, d'un groupe aralkyloxy en C_{6 à 30}, d'un groupe alcaryle en C_{1 à 30}, d'un groupe alcoxyaryle en C_{1 à 30} et d'un groupe hydroxyle ;
où R² est un radical alkylène divalent ayant de un à dix atomes de carbone ;
où chaque R³ est indépendamment choisi dans le groupe constitué d'un groupe alkyle en C_{1 à 30}, d'un groupe aryle en C_{5 à 30}, d'un groupe aralkyle en C_{6 à 30} et d'un groupe alcaryle en C_{1 à 30} ;
où Q est un radical monovalent choisi dans le groupe constitué par -NR⁴₂ et -NR⁴(CH₂)*ₓ*NR⁴₂ ;
où chaque R⁴ est indépendamment choisi dans le groupe constitué d'un hydrogène et d'un groupe alkyle en C_{1 à 4} ;
où x va de 2 à 6 ;
où z est 0 ou 1 ;
où *n* va de 25 à 3000 ; et
où m va de 0 à 3000 ;
à condition qu'au moins 50 % en moles du nombre total de groupes R¹ et R³ soient du méthyle et à condition que lorsque m est 0, z est 1 ;
(c) 5 à 75 % en poids d'un alkyle silane cosmétiquement acceptable de formule (II) 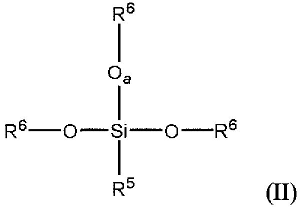
où a est choisi entre 0 et 1 ;
où R⁵ est un groupe alkyle en C_{2 à 10} avec un groupe fonctionnel de réaction d'addition choisi parmi un groupe époxyde et un groupe vinyle ; et
où chaque R⁶ est choisi indépendamment parmi un groupe alkyle en C₁ à ₄ ; et
(d) 15 à 93,8 % en poids d'un polydiméthylsiloxane cosmétiquement acceptable de formule (III) 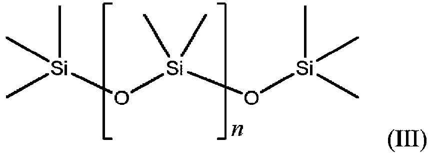 où *n* est une moyenne de 0 à 150.

2. Mélange d'aminosiloxane/alkylsilane selon la revendication 1, où le groupe fonctionnel de réaction d'addition est un groupe époxyde.

3. Mélange d'aminosiloxane/alkylsilane silane selon la revendication 1, où le catalyseur de condensation cosmétiquement acceptable comporte un titantate.

4. Mélange d'aminosiloxane/alkylsilane selon la revendication 1, où z est 0.

5. Mélange d'aminosiloxane/alkylsilane selon la revendication 4, où R² est choisi dans le groupe constitué par le triméthylène, le tétraméthylène,
le pentaméthylène, -CH₂CH(CH₃)CH₂- et -CH₂CH₂CH(CH₃)CH₂-.

6. Mélange d'aminosiloxane/alkylsilane selon la revendication 1, où a est 1.

7. Mélange d'aminosiloxane/alkylsilane selon la revendication 1, où le mélange d'aminosiloxane/alkyl comprend :
1 to 8 % en poids du catalyseur de condensation cosmétiquement acceptable ;
2 à 15 % en poids de l'aminosiloxane cosmétiquement acceptable de formule (I) ;
40 à 60 % en poids de l'alkylsilane cosmétiquement acceptable de formule (II) ; et
25 à 57 % en poids du polydiméthylsiloxane cosmétiquement acceptable de formule (III).

8. Mélange d'aminosiloxane/alkylsilane selon la revendication 7, où le catalyseur de condensation cosmétiquement acceptable est un titanate organique ; où z est 0 et où le groupe fonctionnel de réaction d'addition est choisi dans le groupe constitué d'un groupe époxyde et d'un groupe vinyle.

9. Mélange d'aminosiloxane/alkylsilane selon la revendication 8, dans lequel R² est choisi dans le groupe constitué par le triméthylène, le tétraméthylène,
le pentaméthylène, -CH₂CH(CH₃)CH₂- et -CH₂CH₂CH(CH₃)CH₂-.

10. Mélange d'aminosiloxane/alkylsilane selon la revendication 9, où a est 1.
